# EUROPÄISCHE PATENTANMELDUNG

(11) **EP 1 283 517 A2**
(43) Veröffentlichungstag der Anmeldung: **12.02.2003**
(21) Anmeldenummer: 02017188.0
(22) Anmeldetag: 31.07.2002
(51) Int. Cl.: G10K 15/04, B06B 1/06, G01H 3/12

(54) **Fokussierender elektroakustischer Wandler und Verfahren zum Test seiner Ausgangsleistung**

(30) Priorität: 06.08.2001 DE 10138434
(71) Anmelder: Richard Wolf GmbH, 75438 Knittlingen (DE)
(72) Erfinder: Bauer, Edgar, 76703 Kraichtal (DE)
(74) Vertreter: Wilcken, Thomas, Dipl.-Ing.

(57) **Zusammenfassung**

Bei einem fokussierenden elektroakustischen Wandler mit einem Träger (3), der auf seiner Vorderseite (V) mit einer ersten Gruppe (5) und auf seiner Rückseite (R) mit einer zweiten Gruppe (6) keramischer Piezoelemente (P1, P2) bestückt ist, wird in einer Wandlertestbetriebsart eine Überprüfung der Wandlerleistung dadurch ausgeführt, dass eine der Elementengruppen (5 oder 6) auf der Vorder- oder Rückseite (V oder R) mit einem der bei normalem Betrieb des Wandlers erzeugten Hochspannung entsprechenden Hochspannungsimpuls (Up) beaufschlagt wird und daraufhin ein dabei durch eine über den Träger (3) übertragene mechanische Belastung von den Piezelementen der anderen Elementengruppe (6 oder 5) erzeugter und als Messspannung dienender sekundärer Spannungsimpuls (Us) als Maß für die aktuelle Wandlerleistung der angesteuerten Elementengruppe mit vorgegebenen, zuvor ermittelten und abgespeicherten Referenzwerten der Wandlerleistung der angesteuerten Elementengruppe verglichen wird (Fig. 1).

## Beschreibung

Die Erfindung geht aus von einem fokussierenden elektroakustischen Wandler mit einem Träger, der auf seiner Vorderseite mit einer ersten Gruppe und auf seiner Rückseite mit einer zweiten Gruppe keramischer Piezoelemente bestückt ist, wobei im normalen Betrieb die erste Elementengruppe im Verhältnis zur zweiten Elementengruppe zeitverzögert mit jeweils einem Hochspannungsimpuls ansteuerbar und zur Schallabgabe in Betrieb setzbar ist.

Bei Stoßwellentherapiegeräten muss die Ausgangsleistung des elektroakustischen Wandlers innerhalb bestimmter Zeitintervalle überprüft werden. Bei derartigen extrakorporalen Therapiegeräten wird die Leistung des elektroakustischen Wandlers üblicherweise durch eine Hydrophon-Messung im Wasserbad überprüft. Mit einem gegenüber dem Wandler im Wasser angeordneten und zur Erfassung der erzeugten Stoßwelle eingerichteten Messmikrofon wird der Spitzendruck im Fokus des Wandlers gemessen und der gemessene Wert mit früheren Messungen verglichen. Eine Hydrofon-Messung ist relativ aufwändig und hat den Nachteil, dass sie nur durch Fachpersonal durchgeführt werden kann.

In der Patentschrift DE 41 02 551 C2 ist eine Messanordnung beschrieben, welche einen Reflexionskörper in das Schallfeld des elektroakustischen Wandlers einbringt, um über das reflektierte Signal, welches wiederum im Wandler eine Spannung generiert, die Schallleistung zu bestimmen. Bei diesem Messverfahren besteht der Nachteil, dass der Reflexionskörper exakt im Schallfeld justiert werden muss. Ein weiterer Nachteil ist die relativ kleine Spannung, die durch den reflektierten Schall in dem zu messenden elektroakustischen Wandler erzeugt wird, da sie an den Leitungen desselben gemessen werden muss, welche noch kurz davor mit einer Hochspannung von mehreren kV beaufschlagt wurden und zu dem Zeitpunkt der Messung noch mit Störsignalen behaftet sind.

Gemäß einem im Gebrauchsmuster DE 298 23 797 U beschriebenen Verfahren wird ein Messwandler, der schallempfangsseitig eine räumlich gekrümmte Fläche aufweist, so in das vom zu prüfenden elektroakustischen Wandler erzeugte Schallfeld eingesetzt, dass die Symmetrieachsen des Schallerzeugers und des Messwandlers auf einer durch ihre beiden Fokusse verlaufenden Symmetrielinie liegen. Dadurch können vom Messwandler Messsignale, die die zu messende Schallleistung betreffen, abgeleitet werden, die sich als Nutzsignal weit von den Störsignalen abheben. Auch bei dieser Messanordnung muss der Messwandler an die Geometrie des elektroakustischen Wandlers angepasst und gleichzeitig exakt in dessen Schallfeld eingepasst sein.

Es ist Aufgabe der Erfindung, einen fokussierenden elektroakustischen Wandler und ein Verfahren zum Test seiner Ausgangsleistung anzugeben, wobei die zuvor geschilderten Nachteile des Standes der Technik vermieden und eine exakte Überprüfung der Ausgangsleistung des Wandlers ohne einen in dessen Schallfeld eingebrachten Messwandler ermöglicht werden soll.

Diese Aufgabe wird durch die in den unabhängigen Ansprüchen 1 und 4 angegebenen Merkmale gelöst.

Dadurch, dass der elektroakustische Wandlerin einer Wandlertestbetriebsart dazu eingerichtet ist, eine der Elementengruppen mit einem dem bei normalen Betrieb erzeugten Hochspannungsimpuls entsprechenden primären Hochspannungsimpuls anzusteuern und daraufhin ein dabei durch eine über den Träger übertragene mechanische Belastung von den Piezoelementen der anderen Elementengruppe erzeugter Spannungsimpuls als Messsignal zu empfangen und als Maß für die aktuelle Wandlerleistung mit vorgegebenen, zuvor ermittelten und abgespeicherten Referenzwerten der Wandlerleistung zu vergleichen, kann eine Überprüfung der Wandlerleistung erfolgen, bei der nur eine Elementengruppe auf der Vorder- oder Rückseite mit einem Hochspannungsimpuls angesteuert wird und der an der gegenüberliegenden Elementengruppe erzeugte sekundäre Spannungsimpuls gemessen und ausgewertet wird.

Bei dem erfindungsgemäßen Wandler werden die ermittelten Daten im Speicher eines Microcontrollers abgelegt und können somit jederzeit mit aktuelleren Messungen verglichen werden. Hiermit kann festgestellt werden, ob die Wandlerleistung etwa bedingt durch Schädigungen des Wandlers nachlässt.

Dazu weist der erfindungsgemäße Wandler zweckmäßigerweise einen Microcontroller, einen durch den Microcontroller ansteuer- und einstellbares, zur Erzeugung der Hochspannung eingerichtetes Netzteil, eine Schalteinrichtung zum impulsförmigen Aufschalten der vom Netzteil erzeugten Hochspannung auf die jeweilige Elementengruppe über jeweils eine Leitung, eine vom Microcontroller ansteuerbare Triggereinrichtung, die zur Erzeugung eines Triggersignals eingerichtet ist, das zum Schalten der Schalteinrichtung zugeführt wird, und einen A/D-Wandler auf, der eingangsseitig mit mindestens einer der Leitungen und ausgangsseitig mit dem Microcontroller verbunden ist, wobei die in der Wandlertestbetriebsart von einer Elementengruppe erzeugte Messspannung vom A/D-Wandler digitalisiert und an den Microcontroller übermittelt wird, die dazu eingerichtet ist, die digitalisierte und abgespeicherte Messspannung mit den in ihr früher abgespeicherten Referenzwerten zu vergleichen und auf einem Display das Vergleichsergebnis zur Anzeige zu bringen.

Zweckmäßigerweise sind die Schalteinrichtung und der A/D-Wandler so an die Leitungen von der ersten und zweiten Elementengruppe angekoppelt bzw. durch die Triggereinrichtung anschaltbar, dass die Ansteuerung mit dem primären Hochspannungsimpuls und die Ableitung der sekundären Messspannung jeweils zwischen der ersten und zweiten Elementengruppe abwechselbar sind.

Ein die obige Aufgabe lösendes erfindungsgemäßes Verfahren zum Test der Ausgangsleistung eines fokussierenden elektroakustischen Wandlers, der auf seiner Vorderseite mit einer ersten Gruppe und auf seiner Rückseite mit einer zweiten Gruppe keramischer Piezoelemente bestückt ist, wobei im normalen Betrieb die erste Elementengruppe im Verhältnis zur zweiten Elementengruppe zeitverzögert mit jeweils einem Hochspannungsimpuls ansteuerbar und zur Schallabgabe in Betrieb setzbar ist, zeichnet sich durch folgende Schritte aus:
A eine der Elementengruppen wird mit einem primären Hochspannungsimpuls angesteuert, der einem im normalen Betrieb des elektroakustischen Wandlers erzeugten Hochspannungsimpuls entspricht;
B ein auf den in Schritt A erzeugten primären Hochspannungsimpuls in den Piezoelementen der anderen Elementgruppe durch eine über den Träger übertragene mechanische Belastung derselben erzeugter sekundärer Spannungsimpuls wird als Messsignal abgeleitet, und
C der im Schritt B erzeugte und als Messsignal abgeleitete sekundäre Spannungsimpuls wird mit mindestens einem vorgegebenen, zuvor ermittelten Referenzwert der Wandlerleistung der angesteuerten Elementengruppe verglichen.

Zweckmäßigerweise werden drei bei vorangehenden Messungen des intakten elektroakustischen Wandlers ermittelte Referenzwerte für die Wandlerleistung abgespeichert und zwar solche Normalwerte, die jeweils der maximalen Stauchung, der maximalen Auslenkung und der der maximalen Stauchung nächstfolgenden Stauchung der Piezoelemente derjenigen Elementengruppe entsprechen, von der die sekundäre Messspannung abgeleitet wird.

Durch den erfindungsgemäßen elektroakustischen Wandler und das erfindungsgemäße Verfahren zum Test der Ausgangsleistung desselben können folgende Veränderungen bzw. Schädigungen des elektroakustischen Wandlers erfasst werden:
- Leistungsabfall an der Piezokeramik, bedingt durch mechanische Beschädigungen, Alterung u.s.w.;
- Schadhafte Klebestellen, insbesondere von der Piezokeramik an der als Träger dienenden Wandlerkalotte;
- von der Wandlerkalotte abgelöste Piezoelemente;
- abgelöste Lötpunkte oder Verbindungsdrähte, und
- Veränderungen an der Vergussmasse, die ein Nachlassen der Haftung zur Piezokeramik u.s.w. bewirken können.

Nachfolgend sind Ausführungsbeispiele eines erfindungsgemäßen Wandlers und eines Testverfahrens bezogen auf die Zeichnung beschrieben. Die Zeichnungsfiguren zeigen im einzelnen:
- Fig. 1:: schematisch einen erfindungsgemäßen Wandler mit der blockdiagrammartig eingezeichneten Wandleransteuer- und Testschaltung,
- Fig. 2:: ein Flussdiagramm zur Veranschaulichung einzelner Schritte des erfindungsgemäßen Testverfahrens und
- Fig. 3a und 3b:: grafisch den zeitlichen Verlauf eines mit dem erfindungsgemäßen Testverfahren erfassbaren sekundären Spannungs-impulses jeweils bei einem intakten und einem beschädigten elektroakustischen Wandler.

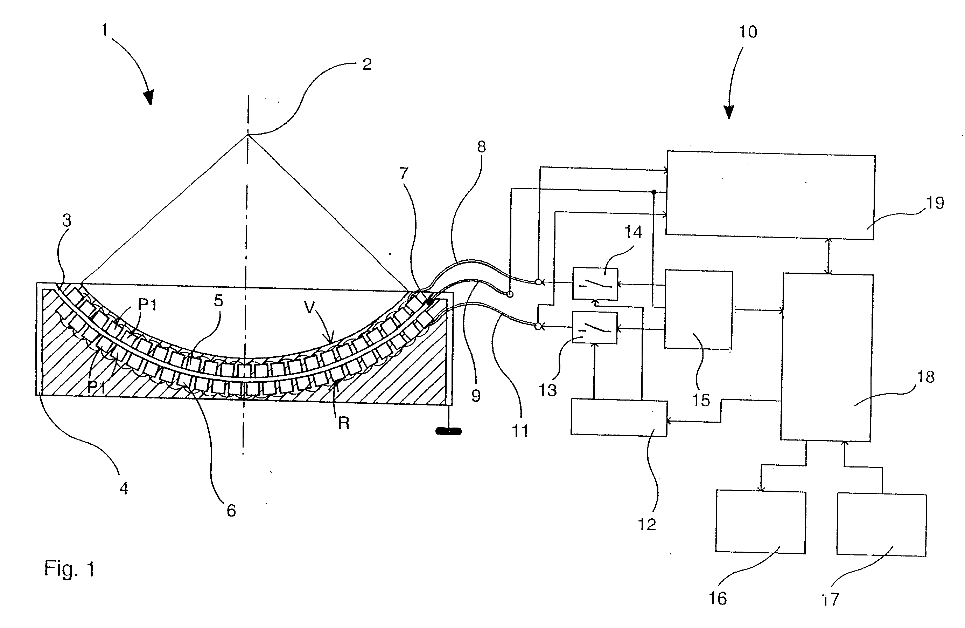

Der in der linken Hälfte der Fig. 1 dargestellte fokussierende elektroakustische Wandler 1, dessen grundsätzlicher Aufbau in der DE 197 33 233 C1 beschrieben ist, weist einen leitenden kalottenförmigen Träger 3 auf, der einstückig mit einem Rohrabschnitt 4 ausgebildet ist, mit dem der Wandler innerhalb einer Geräteanordnung, z.B. eines Therapiegerätes, festgelegt ist. Der kalottenförmige Träger 3 weist auf seiner zum Wandlerfokus 2 weisenden Vorderseite V eine erste Elementengruppe 5 aus keramischen Piezoelementen P1 und auf seiner davon abgewendeten Rückseite R eine zweite Elementengruppe 6 aus keramischen Piezoelementen P2 auf. Die Keramikelemente P1, P2 sind mit einer ihrer Stirnseiten jeweils mit der Vorderseite bzw. Rückseite des Trägers 3 elektrisch leitend verbunden und daran festgelegt. Die freien Stirnseiten der Keramikelemente P1 , P2 sind durch dünne Drähte miteinander verbunden. Die Zwischenräume der vorderseitigen und rückseitigen Keramikelemente P1, P2 sind mit einem schräg schraffierten hochspannungsfesten Isolationsmaterial ausgefüllt, das die Elemente P1, P2 auch an ihren freien Stirnseiten einschließt.

Von der in Fig. 1 rechts gezeichneten Seite des Wandlers führen Verbindungsleitungen 8, 9, 11 zu einer Schaltungsanordnung 10, die zur Durchführung einer normalen Betriebsart und der nachstehend erläuterten Wandlertestbetriebsart eingerichtet ist. Die Zuleitung 8 ist mit den miteinander verbundenen freien Stirnseiten der auf der Vorderseite V des Trägers 3 liegenden ersten Elementengruppe 5 und die Zuleitung 11 mit den freien elektrisch miteinander verbundenen Stirnseiten der auf der Rückseite R des Trägers 3 liegenden zweiten Elementengruppe 6 verbunden, während die Zuleitung 9 mit dem elektrisch leitenden Träger 3 verbunden ist.

Die Schaltungsanordnung 10 weist folgenden Teile auf: einen Microcontroller MCU 18, ein vom Microcontroller 18 ansteuer- und einstellbares Hochspannungsnetzteil 15 zur Erzeugung von Hochspannung, eine Hochspannungsschalteinrichtung mit zwei Schaltern 13, 14 zur impulsförmigen Aufschaltung der vom Hochspannungsnetzteil 15 erzeugten Hochspannung auf die erste und zweite Elementengruppe 5, 6 jeweils über die Leitung 8 und 11, eine Triggereinrichtung 12, die vom Microcontroller 18 ansteuerbar ist und ein Triggersignal erzeugt, das zum Schalten der Schalteinrichtung 13, 14 zugeführt wird, und einen eingangsseitig mit den Leitungen 8 und 11 verbundenen A/D-Wandler 19, der ausgangsseitig mit dem Microcontroller 18 verbunden ist. Ferner zeigt Fig. 1, dass die Schaltungsanordnung 10 eine mit dem Microcontroller 18 verbundene Displayeinheit und eine Bedieneinheit 17 aufweist. An beliebiger Stelle der Trägerkalotte 3 des elektroakustischen Wandlers 1 kann ein Temperatursensor 7 angebracht sein, dessen Funktion weiter unten erläutert wird.

Im Normalbetrieb wird der Wandler 1 wie folgt angesteuert. An der Bedieneinheit 17 wird die gewünschte Stoßwellenintensität eingestellt, woraufhin der Microcontroller 18 am Hochspannungsnetzteil 15 die entsprechende Hochspannung einstellt. Erfolgt nun eine Stoßwellenfreigabe an der Bedieneinheit 17, so gibt der Microcontroller 18 die Triggereinrichtung 12 frei, deren Triggersignal unmittelbar danach den Hochspannungsschalter 13 und nach einer bestimmten Verzögerungszeit den Hochspannungsschalter 14 zur Erzeugung eines Hochspannungsimpulses auslöst. Über die Hochspannungsschalter 13 und 14 gelangt der Hochspannungsimpuls vom Netzteil 15 zu den Hochspannungsanschlüssen 8 und 11, die mit den jeweiligen keramischen Piezoelementen P1 und P2 auf der Vorder- und Rückseite V, R verbunden sind.

Erfindungsgemäß erfolgt nun eine Überprüfung oder ein Test der Wandlerleistung, indem nur eine Elementengruppe 5 oder 6 auf der Vorder- oder Rückseite V, R mit einem Hochspannungsimpuls angesteuert wird, der dem im normalen Betrieb erzeugten Hochspannungsimpuls entspricht. Daraufhin wird an der gegenüberliegenden Seite, d.h. an der anderen nicht angesteuerten Elementengruppe, der sekundäre Spannungsimpuls gemessen, der durch die Übertragung des mechanischen Stoßes von der angesteuerten Elementengruppe über den Träger 3 zur nicht angesteuerten Elementengruppe erzeugt wird.

Zur Durchführung dieses Wandlertests wird der in der Schaltungsanordnung 10 vorhandene Microcontroller und der mit ihm verbundenen A/D-Wandler 19, der an mindestens einer Hochspannungsanschlussleitung des Wandlers 1 oder, wie in Fig. 1 gezeigt, an beiden Hochspannungsanschlussleitungen 8 und 11 des Wandlers 1 angeschlossen ist, verwendet.

Vor der Durchführung des praktischen Wandlertests werden z.B. herstellerseitig bei einem intakten bzw. neuen Wandler 1 mit demselben Verfahren die entsprechneden Spannungswerte gemessen und als Referenzwerte im Speicher des Microcontrollers 18 abgelegt und können dadurch jederzeit mit aktuell ermittelten Messspannungswerten verglichen werden. Mit diesem Vergleich kann festgestellt werden, ob die Wandlerleistung bedingt durch Schädigungen des Wandlers nachlässt.

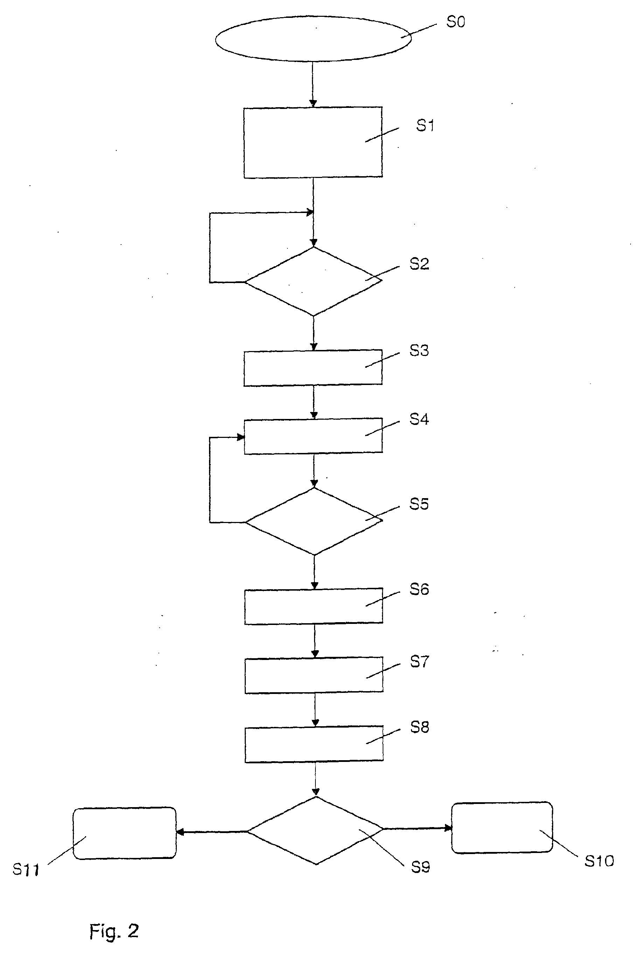

Nachstehend werden einzelne Schritte eines mit einem erfindungsgemäßen Verfahren bei einem derartigen elektroakustischen Wandler ausgeführten Wandlertests an Hand des in Fig. 2 dargestellten Flussdiagramms näher erläutert:

In der Bedieneinheit 17 des Therapiegeräts wird im Menü das Programm "Wandlertest" angewählt und dadurch ein automatischer Testzyklus gestartet (Schritt S0).

Der Microcontroller 18 stellt am Hochspannungsnetzteil 15 die Spannung ein, mit welcher der Wandler 1 angesteuert werden soll (Schritt S1).

Die MCU 18 wartet auf eine Stoßwellenfreigabe, die über einen Handtaster an der Bedieneinheit 17 ausgelöst wird (Schritt S2).

Nach erfolgter Stoßwellenfreigabe löst der Microcontroller 18 in der Triggereinrichtung 12 den Trigger für den Hochspannungsschalter aus (Schritt S3), wodurch der Hochspannungsschalter (z. B. Schalter 13) betätigt, d.h. geschlossen (Schritt S4) wird.

Dieser Hochspannungsschalter bleibt für eine bestimmte Zeitdauer (z.B. 10 µs) eingeschaltet (Schritt S5).

Anschließend wird der Hochspannungsschalter wieder geöffnet (Schritt S6).

Der Wandler 1 wurde nun kurzzeitig auf der Rückseite R mit dem Hochspannungsimpuls (primärer Spannungsimpuls Up) beaufschlagt. Der dadurch von der Elementengruppe 6 auf der Rückseite des Trägers 3 erzeugte mechanische Stoßimpuls wird über den Träger 3 und die Vergussmasse auf die auf der Vorderseite V befindliche Elementengruppe 5 übertragen, woraufhin an der Hochspannungsanschlussleitung 8, die mit der auf der Vorderseite V befindlichen Elementengruppe 5 verbunden ist, ein Spannungsimpuls (sekundärer Spannungsimpuls Us) abgreifbar ist.

Diesersekundäre Spannungsimpuls wird vom A/D-Wandler 19 in ein digitales Signal umgesetzt und in den Microcontroller 18 eingelesen (Schritt S7). Dann folgt ein optionaler Schritt S8, bei dem der Microcontroller 18 den Temperaturwert des Wandlers 1 mittels des Temperatursensors 7 einliest.

Im Schritt S9 vergleicht der Microcontroller 18 die vom A/D-Wandler 19 eingelesenen digitalisierten Werte des sekundären Spannungsimpulses Us mit den zuvor im Speicher als Referenzwerte abgelegten entsprechenden Werten, wie sie zuvor an Hand einer Leistungstestmessung des neuen Wandlers eingespeichert wurden. Nach der Bewertung durch den Microcontroller 18 kann diese dem Display 16 eine Anzeige ausgeben, z.B. "Wandlerleistung in Ordnung" (Schritt S10) oder"Wandlerleistung zu niedrig" (Schritt S11).

Die in Fig. 1 dargestellte Schaltungsanordnung 10 ist in Verbindung mit dem elektroakustischen Wandler 1 auch so eingerichtet, dass zuerst die auf der Vorderseite V des Wandlers 1 befindliche erste Elementengruppe 5 mit dem primären Hochspannungsimpuls Up durch Schließen des Schalters 14 angesteuert wird und die Messung des sekundären Spannungsimpulses Us von der auf der Rückseite R angeordneten zweiten Elementengruppe 6 erfolgt. Hierzu ist die Hochspannungsanschlussleitung 11 ebenfalls an den A/D-Wandler 19 geführt. Auf diese Weise kann die Wandlertestmessung zeitlich nacheinander abwechselnd auf jeder Wandlerseite V, R durchgeführt werden, was die Gültigkeit und Genauigkeit der Messergebnisse noch verbessert.

Die in Fig. 2 mit dem Schritt S8 beschriebene Überwachung der Temperatur des elektroakustischen Wandlers 1 mit Hilfe des Temperatursensors 7 und die anschließende Messwertanpassung ist lediglich ein optionales Ausführungsbeispiel. Normalerweise würde es ausreichen, die in Betracht kommenden Amplituden des sekundären Spannungsimpulses bei verschiedenen Temperaturen zu ermitteln und die hieraus erhaltenen Kennlinien im Microcontroller 18 abzuspeichern, wodurch bei jeder Messung eine Messwertanpassung erfolgen kann. Es ist jedoch möglich, dass die Temperaturkennlinie nicht für jeden Wandler gleich ist, da sie von den für den Wandler 1 verwendeten Materialien und von der Bestückungszahl der Piezoelemente abhängig ist. Hierzu müssten Tests mit zwei Wandlern durchgeführt werden, die das obere und untere Maximum der Bestückungsdichte aufweisen. Gibt es dabei eine große Differenz der Temperaturkennlinien, müsste die dem Wandler zugehörige Kennlinie jeweils im Speicher abgelegt werden. Wenn, um den obigen Aufwand kleiner zu halten, der Temperatursensor lediglich zur Überwachung der Temperatur eingesetzt ist, kann der Microcontroller 18 mit der vom Temperatursensor 7 gemessenen Temperatur des Wandlers 1 die Entscheidung treffen, ob eine Testleistungsmessung, d.h. Messung des sekundären Spannungsimpulses Us, durchgeführt werden kann oder nicht.

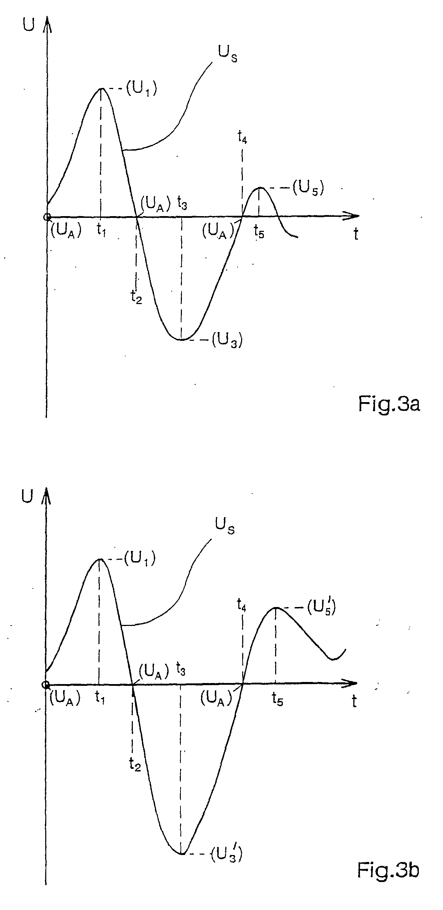

Fig. 3a zeigt grafisch einen typischen Spannungsverlauf des sekundären Spannungsimpulses Us eines intakten bzw. neuen elektroakustischen Wandlers 1, wobei der sekundäre Spannungsimpuls Us an der Hochspannungsanschlussleitung 8 der vorderseitigen Elementengruppe bei Hochspannungsansteuerung der rückseitigen Elementengruppe 6 gemessen wurde. Zum Zeitpunkt t0 befindet sich das Piezoelement in Ruhelage (Ruhespannung U_{A}). Das erste Spannungsmaximum U1 zum Zeitpunkt t1 gibt den Spannungswert bei maximaler Stauchung des Elements in Folge der Druckwelle an. Anschließend durchläuft das Element zum Zeitpunkt t2 die Ausgangslage U_{A}. Zum Zeitpunkt t3 ist das Element maximal ausgelenkt und hat ein negatives Spannungsmaximum U3. Dann durchläuft zum Zeitpunkt t4 das Element wieder die Ausgangslage U_{A}. Zum Zeitpunkt t5 zieht sich das Element wieder zusammen, die Spannungsamplitude U5 erreicht jedoch nicht mehr die Höhe der Amplitude U1 zum Zeitpunkt t1.

Fig. 3b zeigt grafisch den Spannungsverlauf des sekundären Spannungsimpulses Us bei einem beschädigten Wandler mit Druckverlust, der durch die Vergussmasse bedingt ist. Der Spannungsverlauf gemäß Fig. 3b zeigt, dass die Amplitude U3' zum Zeitpunkt t3 und die Amplitude U5' zum Zeitpunkt t5 größer sind, weil durch Rissbildung der Vergussmasse oder durch Ablösen der Vergussmasse vom Piezoelement die mechanische Kraft der Vergussmasse auf das Piezoelement nachlässt.

Die Amplitude U1 zum Zeitpunkt t1 muss jedoch noch ihren Maximalwert, wie in Fig. 3a, haben. Nimmt die Amplitude U1 ab, wäre der Wandlerschaden auf eine Beschädigung von Piezoelementen oder deren Kontaktierung zurückzuführen.

Um den Test der Wandlerleistung und dessen Unversehrtheit durchzuführen, ist es zu bevorzugen, wenn zuvor mehrere Referenzwerte am neuen oder intakten Wandler gemessen und gespeichert werden. Dazu werden durch Messungen am intakten oder neuen elektroakustischen Wandler mindestens die drei Amplitudenwerte U1, U3 und U5 im Speicher abgelegt, um dann mit entsprechenden aktuell gemessenen Spannungsamplitudenwerten verglichen zu werden.

## Patentansprüche

1. Fokussierender elektroakustischer Wandler mit einem Träger (3), der auf seiner Vorderseite (V) mit einer ersten Gruppe (5) und auf seiner Rückseite (R) mit einer zweiten Gruppe (6) keramischer Piezoelemente (P1, P2) bestückt ist, wobei im normalen Betrieb des elektroakustischen Wandlers die erste Elementengruppe (5) im Verhältnis zurzweiten Elementengruppe (6) zeitverzögert mit jeweils einem Hochspannungsimpuls ansteuerbar und zur Schallabgabe in Betrieb setzbar ist, **dadurch gekennzeichnet, dass** der Wandler für einen Wandlertestbetrieb so eingerichtet ist, dass eine der Elementgruppen (5 oder 6) mit einem dem beim normalen Betrieb erzeugten Hochspannungsimpuls entsprechenden primären Hochspannungsimpuls (Up) ansteuerbar ist und daraufhin ein dabei durch eine über den Träger (3) übertragene mechanische Belastung von den Piezoelementen der anderen Elementengruppe (6 oder 5) erzeugter sekundärer Spannungsimpuls (Us) als Messsignal erzeugbarund als Maß fürdie aktuelle Wandlerleistung mit vorgegebenen, zuvor ermittelten Referenzwerten, die die normale Wandlerleistung angeben, vergleichbar ist.

2. Wandler nach Anspruch 1, **dadurch gekennzeichnet, dass** er weiterhin aufweist: einen Microcontroller (18), ein durch den Microcontroller (18) ansteuer- und einstellbares und zur Erzeugung der Hochspannung eingerichtetes Netzteil (15), eine Hochspannungsschalteinrichtung (13, 14) zum impulsweisen Aufschalten des vom Netzteil (15) erzeugten Hochspannungsimpulses auf jede Elementengruppe (5, 6) über jeweils eine Anschlussleitung (8, 11), einen vom Microcontroller (18) ansteuerbare Triggereinrichtung (12) zur Erzeugung eines Triggersignals, das zum Schalten der Schalteinrichtung (13, 14) zugeführt wird und einen A/D-Wandler (19), der eingangsseitig mit mindestens einer der Anschlußleitungen (8, 11) und ausgangsseitig mit dem Microcontroller (18) verbunden ist, wobei der in der Wandlertestbetriebsart von einer Elementengruppe (5, 6) abgegriffene sekundäre Spannungsimpuls (Us) vom A/D-Wandler (19) digitalisiert und an den Microcontroller übermittelt wird, der dazu eingerichtet ist, die digitalisierte Spannung zu speichern und mit den in ihm zuvor abgespeicherten Referenzwerten zu vergleichen und auf einem Display (16) das Vergleichsergebnis zur Anzeige zu bringen.

3. Wandler nach Anspruch 1 oder 2, **dadurch gekennzeichnet, dass** in der Wandlertestbetriebsart nach der Ansteuerung der einen Elementengruppe mit dem primären Hochspannungsimpuls (Up) und der Ableitung des sekundären Spannungsimpulses (Us) von der anderen Elementengruppe die Ansteuerung mittels der Schalteinrichtung (13, 14) umkehrbar ist, so dass die zuvor nicht angesteuerte Elementengruppe jetzt mit dem primären Hochspannungsimpuls (Up) beaufschlagt und der sekundäre Spannungsimpuls (Us) als Messsignal von der anderen Elementengruppe abgeleitet wird.

4. Verfahren zum Test der Ausgangsleistung eines fokussierenden elektroakustischen Wandlers (1), der auf seiner Vorderseite (V) mit einer ersten Gruppe (5) und auf seiner Rückseite (R) mit einerzweiten Gruppe (6) keramischer Piezoelemente (P1, P2) bestückt ist, wobei im normalen Betrieb die erste Elementengruppe (5) im Verhältnis zur zweiten Elementengruppe (6) zeitverzögert mit jeweils einem Hochspannungsimpuls ansteuerbar und zur Schallabgabe in Betrieb setzbar ist, **dadurch gekennzeichnet, dass** das Verfahren folgende Schritte aufweist:
A eine der Elementengruppen (5 oder 6) wird mit einem primären Hochspannungsimpuls (Up) angesteuert, der einem im normalen Betrieb des elektroakustischen Wandlers erzeugten Hochspannungsimpuls entspricht;
B ein auf den in Schritt A erzeugten primären Hochspannungsimpuls (Up) in den Piezoelementen der anderen Elementengruppe (6 oder 5) durch eine über den Träger (3) übertragene mechanische Belastung derselben erzeugter sekundärer Spannungsimpuls (Us) wird als Messsignal abgeleitet, und der im Schritt B erzeugte und als Messsignal abgeleitete sekundäre Spannungsimpuls (Us) wird mit mindestens einem vorgegebenen zuvor mit den Verfahrensschritten A und B ermittelten Referenzwert der Wandlerleistung der angesteuerten Elementengruppe verglichen.

5. Verfahren nach Anspruch 4, **dadurch gekennzeichnet, dass** der in Schritt B abgeleitete sekundäre Spannungsimpuls (Us) zur Erzeugung des Messsignals einer A/D-Wandlung unterworfen und in ein digitales Messsignal umgewandelt wird.

6. Verfahren nach Anspruch 4 oder 5, **dadurch gekennzeichnet, dass** bei der zuvor ausgeführten Leistungsmessung des intakten elektroakustischen Wandlers drei Spannungsamplituden (U1, U3, U5) des sekundären Spannungsimpulses (Us) als Referenzwerte abgespeichert werden, die jeweils der maximalen Stauchung, der maximalen Auslenkung und der der maximalen Stauchung nächstfolgenden Stauchung der Piezoelemente der anderen Elementengruppe entsprechen.

7. Verfahren nach einem der Ansprüche 4 bis 6, **dadurch gekennzeichnet, dass** die mit dem primären Spannungsimpuls (Up) in Schritt A angesteuerte Elementengruppe gewechselt wird und dass der sekundäre Spannungsimpuls (Us) dann von der jeweils anderen Elementengruppe abgeleitet wird.
